# EUROPEAN PATENT APPLICATION

(11) **EP 4 201 483 A1**
(43) Date of publication of application: **28.06.2023**
(21) Application number: 21217169.8
(22) Date of filing: 22.12.2021
(51) Int. Cl.: A61P 31/04, A61K 38/04, C07K 7/00

(54) **LPL1 DERIVED IMMUNE ENHANCING PEPTIDES**

(71) Applicant: Eberhard-Karls-Universität Tübingen, 72074 Tübingen (DE)
(72) Inventor: Götz, Friedrich, 72074 Tübingen (DE); Ammanath, Aparna Viswanathan, 72074 Tübingen (DE)
(74) Representative: Kuttenkeuler, David

(57) **Abstract**

The invention pertains to immunologically active peptides derived from bacterial lipoprotein-like lipoproteins (Lpl). The invention provides the peptides as therapeutics for the treatment of diseases such as sepsis by enhancing a host's immune system.

## Description

### FIELD OF THE INVENTION

The invention pertains to immunologically active peptides derived from bacterial lipoprotein-like lipoproteins (Lpl). The invention provides the peptides as therapeutics for the treatment of diseases such as sepsis by enhancing a host's immune system.

### DESCRIPTION

*Staphylococcus aureus* is one of the top three pathogens that cause community-acquired, healthcare-related nosocomial infections in humans. It lives as a commensal organism, but it can also infect the body at various sites. Diseases greatly differ, from skin lesions to invasive infections. Twenty to thirty percent of the healthy population is colonized with *S. aureus* in the nostrils, and a substantial percentage of *S. aureus* bacteremia originates from endogenous colonies from the nasal mucosa. The clinical expression of sepsis covers a continuum of manifestations, with the most violent form termed "septic shock." In this state, vascular offense and systemic inflammation lead to endangered cardiac function and blood pressure drops that cause impaired oxygen delivery, organ failure, and death. Sepsis-related mortality and the lack of mitigating clinical approaches attest to our limited understanding of the complex host-*S*. *aureus* interactions. However, also many other bacteria can induce sepsis or similar diseases.

*S. aureus* Lpl protein triggers human host cell invasion via activation of Hsp90 receptor. In *S. aureus* the lpl genes are part of a pathogenicity island and, typically, lpl-homologous genes are aligned in tandem. Since the contribution of the lpl gene cluster to virulence was unclear, the inventors deleted the entire lpl gene cluster in *S. aureus* USA300. The mutant showed not only a decrease in the stimulation of pro-inflammatory cytokines in human monocytes, macrophages and keratinocytes. More important was the finding that the lpl cluster contributed to invasion of *S. aureus* into human keratinocytes and mouse skin and the non-invasive *S. carnosus* expressing the lpl gene cluster became invasive. Additionally, in a murine kidney abscess model the bacterial burden in the kidneys was higher in wild type than in mutant mice indicating that the lpl cluster contributes to virulence (Nguyen et al., 2015).

Indeed, the Lpl protein triggers *S. aureus* internalization by various human epithelial cells and also keratinocytes 3 to 5-fold (Nguyen et al., 2015). The underlaying mechanism is due to the activation of the human heat shock proteins Hsp90α and Hsp90β (Tribelli et al., 2019). Lpli-Hsp90 interaction induces F-actin formation thus triggering an endocytosis-like internalization. Thus, we uncovered a new host cell invasion principle based on Lpl-Hsp90 interaction (Tribelli et al., 2019).

Thus, it is an objection of the invention to provide novel therapeutic treatment options to tackle septic infections and other infectious diseases, for example, by *S. aureus.*

### BRIEF DESCRIPTION OF THE INVENTION

Generally, and by way of brief description, the main aspects of the present invention can be described as follows:

In **a first aspect,** the invention pertains to an isolated peptide, or a peptide-derivative or -analog thereof, wherein the peptide sequence has a length of 6 to 40 amino acids, and wherein the peptide sequence is at least 70% identical to a peptide sequence of 6 to 40 amino acids in a core domain of a bacterial lipoprotein, preferably Lpli.

In **a second aspect,** the invention pertains to a fusion peptide or fusion protein consisting of two sequence portions, wherein the first sequence portion is identical to the sequence of the isolated peptide recited in the first aspect, and wherein the second portion consists of a sequence that is not more than 70%, preferably 50% even more preferably 30% identical to sequence of similar length in a bacterial lipoprotein

In **a third aspect,** the invention pertains to an isolated nucleic acid comprising a nucleic acid sequence encoding for a peptide or protein, wherein the encoded peptide or protein consists of a peptide of the first aspect or consists of a fusion peptide or fusion protein of the second aspect.

In **a fourth aspect,** the invention pertains a vector comprising the nucleic acid of the invention.

In **a fifth aspect,** the invention pertains to a recombinant cell comprising the nucleic acid or vector of the invention.

In **a sixth aspect,** the invention pertains to a pharmaceutical composition comprising (i) the isolated peptide of the invention, or the fusion peptide or fusion protein of the invention, or the nucleic acid of the invention, or the vector of the invention, or the recombinant cell of the invention; and (ii) a pharmaceutically acceptable carrier and/or excipient.

In **a seventh aspect,** the invention pertains to a compound or composition for use in the treatment of an infection in a subject, wherein the compound or composition comprises the isolated peptide of the invention, or the fusion peptide or fusion protein of the invention, or the nucleic acid of the invention, or the vector of the invention, or the recombinant cell of the invention or the pharmaceutical composition of the invention.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **a first aspect,** the invention pertains to an isolated peptide, or a peptide-derivative or -analog thereof, wherein the peptide sequence has a length of 6 to 40 amino acids, and wherein the peptide sequence is at least 70% identical to a peptide sequence of 6 to 40 amino acids in a core domain of a bacterial lipoprotein, preferably a lipoprotein-like lipoprotein (Lpl), such as Lpli.

The invention generally involves the peptide of the first aspect, or any of the described variant thereof, for a use in a method of treating an infection, comprising administering a regimen of such peptide to enhance immune responses of an infected host to pathogen exposure, or potential pathogen exposure to prevent, reduce or treat the infection, in particular sepsis, severe sepsis or septic shock.

In certain preferred embodiments the Lpli protein is derived from a pathogenic microorganism such as of the genus *Staphylococcus, Listeria, Lactobacillus, Escherichia, Klebsiella,* or *Pseudomonas*; and preferably is *S. aureus* Lpli. Sequences of the respective LpL proteins are shown in SEQ ID Nos: 1, and 5 to 12 as provided herein below. The protein sequence *of S. aureus* Lpli is shown herein as SEQ ID NO: 1.

In preferred embodiments of the invention the core domain of the lipoprotein comprises a protein sequence that is at least 70%, preferably 80%, identical to LNC1 (sequence of the LNC1 core of Lpli of *S. aureus*), which is shown herein as SEQ ID NO: 2.

In accordance with the invention, it is preferred that the peptide sequence has a length of 6 to 30 amino acids, preferably of 6 to 20 amino acids, most preferably of 8 to 20 amino acids.

In one preferred embodiment of the invention, the peptide sequence is at least 80%, preferably 85%, 90%, 95% and most preferably 100% identical to a peptide sequence of 6 to 40 amino acids in a core domain of a bacterial lipoprotein, preferably a core domain of Lpli (as shown in SEQ ID NO: 2).

In further preferred embodiments of the invention, the peptide sequence over its full length is at least 80% identical, preferably 85%, 90%, or 100% identical, to a sequence of identical length in LNC1 (sequence of the LNC1 core of Lpli, herein shown as SEQ ID NO: 2).

The isolated peptide the invention, in preferred embodiments may comprise, or preferably consist essentially of, or consist of, a sequence of the formula: X₁X₂X₃X₄X₅X₆X₇X₈X₉X₁₀X₁₁X₁₂X₁₃X₁₄X₁₅, wherein X is an amino acid or no amino acid (absent) according to the following definitions:
- X₁ is absent or T, preferably X₁ is absent if X₂ is absent;
- X₂ is absent or A, preferably X₂ is absent if X₃ is absent;
- X₃ is absent or K;
- X₄ is G;
- X₅ is any amino acid, preferably any amino acid is Y or H;
- X₆ is Y;
- X₇ is F;
- X₈ is V;
- X₉ is any amino acid, preferably any amino acid is T, K or R;
- X₁₀ is T;
- X₁₁ is F;
- X₁₂ is Y;
- X₁₃ is absent or any amino acid, preferably any amino acid is R or N;
- X₁₄ is absent or any amino acid (preferably any amino acid is N or K), preferably X₁₄ is absent if X₁₃ is absent;
- X₁₅ is absent or any amino acid (preferably any amino acid is G or D), preferably X₁₅ is absent if X₁₄ is absent.
- X12 is Y;
- X13 is absent or any amino acid, preferably any amino acid is R or N;
- X14 is absent or any amino acid (preferably any amino acid is N or K), preferably X14 is absent if X13 is absent;
- X15 is absent or any amino acid (preferably any amino acid is G or D), preferably X15 is absent if X14 is absent.

It is understood that the above formula (X₁ to X₁₅) denotes a sequence of consecutive and (directly without any AA in between) adjoining amino acid positions.

The most preferred isolated peptide of the invention as described herein, comprises, or consists (essentially) of, a sequence that is at least 90%, preferably 100%, identical to the peptide sequence as shown herein, in particular in any one of SEQ ID NO: 3, 4 and 13-28; most preferably: GHYFVTTFY (SEQ ID NO: 3) or TAKGHYFVTTFYRNG (SEQ ID NO: 4) wherein herein elsewhere the peptides are also referred to as L13 (SEQ ID NO: 3) and L15 (SEQ ID NO: 4).

It is further preferred that the peptide according to the invention has a maximal length of 16 amino acids, preferably of 15 amino acids, preferably wherein the length of the isolated peptide consists of either 9 or 15 amino acids.

Bacterial proteins and peptides often comprise an N-terminal formyl group, which is known as one of the most conserved features that distinguish eubacteria from archaea and eukaryotes. Hence, in some preferred embodiment the isolated peptide of the invention, or the peptide-derivative or -analog thereof, comprises an N-terminal formylation.

In a further preferred embodiment, the peptide, or the peptide-derivative or -analog thereof, comprises a C-terminal amidation.

In another embodiment which is preferred, the peptide, or the peptide-derivative or - analog thereof, comprises and N-terminal formylation and a C-terminal amidation.

The polypeptides disclosed herein may be reduced to generate mimetics, e.g. peptide or non-peptide agents, which are able to mimic binding of the authentic protein to another cellular partner. Such mutagenic techniques as described above, as well as the thioredoxin system, are also particularly useful for mapping the determinants of a protein which participates in a protein-protein interaction with another protein. To illustrate, the critical residues of a protein which are involved in molecular recognition of a substrate protein may be determined and used to generate peptidomimetics that may bind to the substrate protein. The peptidomimetic may then be used as an inhibitor of the wild-type protein by binding to the substrate and covering up the critical residues needed for interaction with the wild-type protein, thereby preventing interaction of the protein and the substrate. By employing, for example, scanning mutagenesis to map the amino acid residues of a protein which are involved in binding a substrate polypeptide, peptidomimetic compounds may be generated which mimic those residues in binding to the substrate.

For instance, derivatives of the peptides/proteins described herein may be chemically modified peptides and peptidomimetics. Peptidomimetics are compounds based on, or derived from, peptides and proteins. Peptidomimetics can be obtained by structural modification of known peptide sequences using unnatural amino acids, conformational restraints, isosteric replacement, and the like. The subject peptidomimetics constitute the continuum of structural space between peptides and non-peptide synthetic structures; peptidomimetics may be useful, therefore, in delineating pharmacophores and in helping to translate peptides into nonpeptide compounds with the activity of the parent peptides.

In **a second aspect,** the invention pertains to a fusion peptide or fusion protein consisting of two sequence portions, wherein the first sequence portion is identical to the sequence of the isolated peptide recited in the first aspect, and wherein the second portion consists of a sequence that is not more than 70%, preferably 50% even more preferably 30% identical to sequence of similar length in a bacterial lipoprotein

In **a third aspect,** the invention pertains to an isolated nucleic acid comprising a nucleic acid sequence encoding for a peptide or protein, wherein the encoded peptide or protein consists of a peptide of the first aspect or consists of a fusion peptide or fusion protein of the second aspect.

In **a fourth aspect,** the invention pertains to a vector comprising the nucleic acid of the invention.

In **a fifth aspect,** the invention pertains to a recombinant cell comprising the nucleic acid or vector of the invention.

In **a sixth aspect,** the invention pertains to a pharmaceutical composition comprising (i) the isolated peptide of the invention, or the fusion peptide or fusion protein of the invention, or the nucleic acid of the invention, or the vector of the invention, or the recombinant cell of the invention; and (ii) a pharmaceutically acceptable carrier and/or excipient.

In **a seventh aspect,** the invention pertains to a compound or composition for use in the treatment of an infection in a subject, wherein the compound or composition comprises the isolated peptide of the invention, or the fusion peptide or fusion protein of the invention, or the nucleic acid of the invention, or the vector of the invention, or the recombinant cell of the invention or the pharmaceutical composition of the invention.

In further aspects of the invention there is provided a method of treating an infection in a subject, the method comprising the steps of administering a therapeutically effective amount of a peptide or fusion protein, nucleic acid, recombinant cell or composition of the invention to the subject.

In further aspects the invention pertains to a use of a peptide or fusion protein, nucleic acid, recombinant cell or composition of the invention in the manufacturing of a medicament for the treatment of an infection in a subject.

As used herein, the term "infection" refers to an invasion and the multiplication of microorganisms such as *S. aureus* in body tissues, which may be clinically unapparent or result in local cellular injury due to competitive metabolism, toxins, intracellular replication or antigen antibody response. The infection may remain localized, subclinical and temporary if the body's defensive mechanisms are effective. A local infection may persist and spread by extension to become an acute, subacute or chronic clinical infection or disease state. A local infection may also become systemic when the microorganisms gain access to the lymphatic or vascular system.

Accordingly, the term "septic arthritis" as used herein and in the claims includes infections of the joint and surrounding tissues caused by the above listed pathogens as well as any other pathogens having the ability to infect the joint and surrounding tissues. Surrounding tissues include, but are not limited to, surrounding muscle, related tendons, connecting bones, bursae, tendon sheaths, synovium, synovial fluid, and related cartilage.

The term "treating septic arthritis" includes eradication of the pathogens/bacteria causing the underlying infection associated with septic arthritis, inhibition of bacterial growth, reduction in bacterial concentration, reduction in recovery time from infection, improvement, elimination, or reduction of symptoms of infection such as swelling, necrosis, fever, pain, weakness, and or other indicators as are selected as appropriate measures by those skilled in the art.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

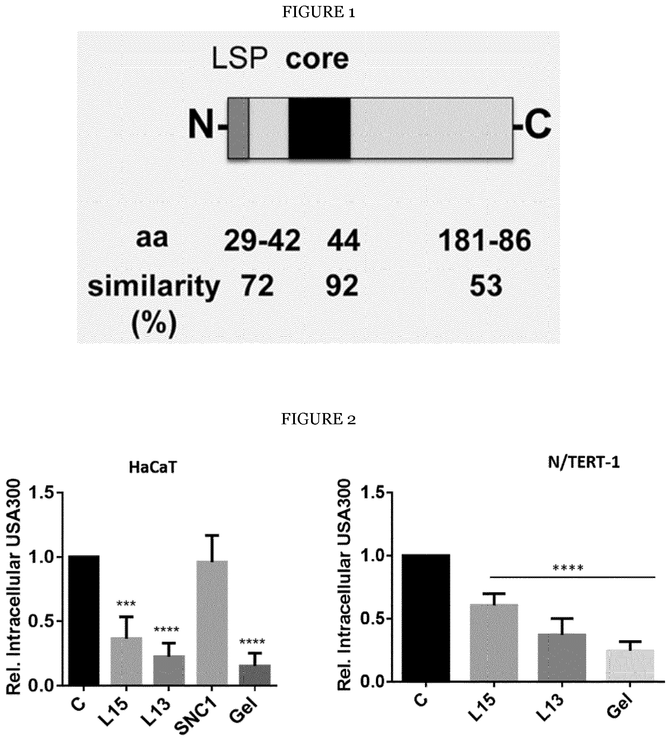

**Figure 1****:** Extent of sequence homology of the nine Lpl proteins encoded by the lpl operon in **S. aureus** USA300. The 44 aa long core region shows 92% of similarity among the paralogs (Nguyen et al., 2015). LSP, leader signal peptide. Total length without LSP is 267 aa. We have chosen Lpli as a prototype of Lpl proteins for further studies.
**Figure 2**: L13 and L15 inhibit *S. aureus* internalization by HaCaT cells and N/TERT1 cells. N/TERT-1 are immortalized human keratinocytes. L15 was used at 20 µM), L13 at 30 µM and geldanamycin at 5 µM. SNC1 is representative for the many peptides that had no effect

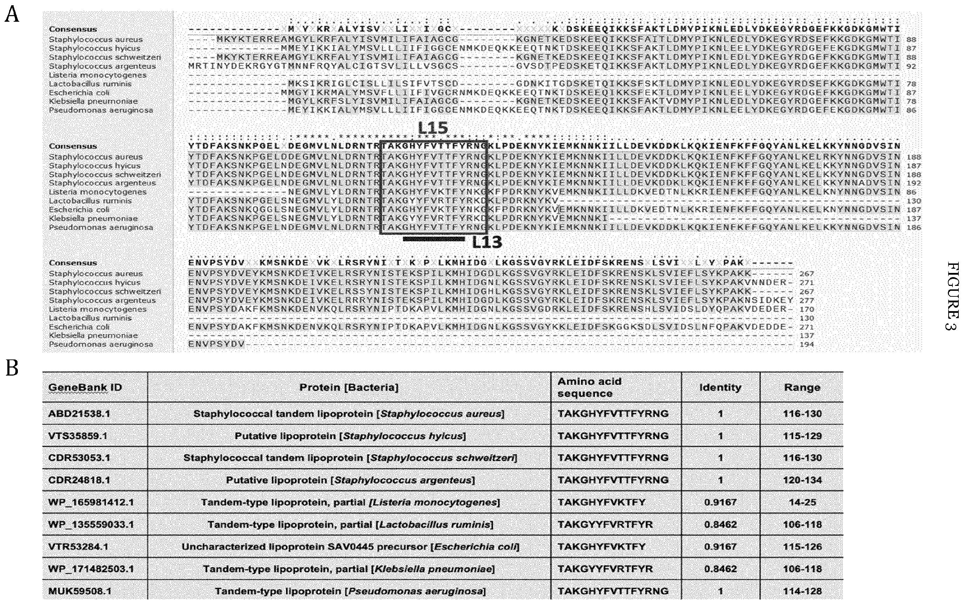

**Figure 3**: Alignment of Lpl protein homologs. (**A**) Multiple sequence alignment of Lpls generated by using SnapGene software. Amino acids that match the Lpli sequence are highlighted in yellow. The L13 (**underlined**) and L15 (box) sequences are highly conserved (**B**) Comparative analyses of amino acid sequences of L15 obtained from Lpli *of S. aureus* USA300 compared to corresponding orthologues in other species (*Staphylococcus aureus, Staphylococcus hyicus, Staphylococcus schweitzeri, Staphylococcus argenteus, Listeria monocytogenes, Lactobacillus ruminis, Escherichia coli, Klebsiella pneumoniae, Pseudomonas aeruginosa).*

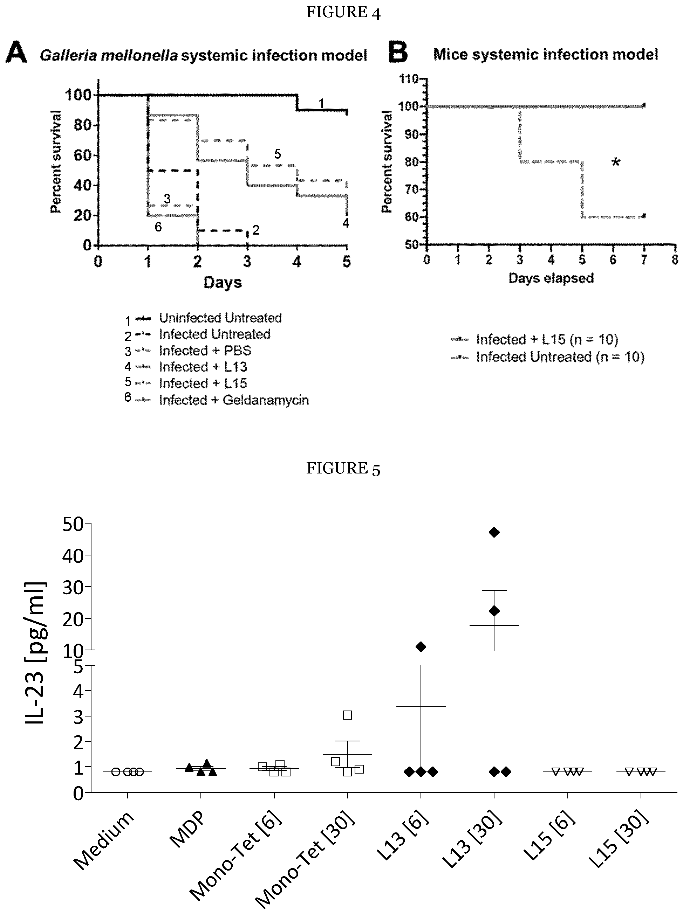

**Figure 4:** A) In vivo systemic infection models of Galleria mellonella (n=30) and B) septic arthritis mouse model (n=10). The organisms (larvae and mice) were pretreated with peptides and the controls and then infected with *S. aureus.* There was a significant improvement in the mortality rates of larvae pretreated with L13 (30 µM) and L15 (20 µM), while geldanamycin (10 µM) further reduced survival. (B) In the septic arthritis model only L15 was used, as L13 was less soluble. In this sepsis model 100% of the mice survived when pretreated with L15 one hour before infection. Without treatment only 60 % of the mice survived. An interesting side observation was that the mice were hyperactive when treated with L15. A therapeutic model (treatment at intervals post-infection) is already envisaged in the course of this project.
**Figure 5****:** Quantification of the secreted cytokine IL-23 after stimulation of peripheral blood mononuclear cells of different donors with various cell wall components of *Staphylococcus aureus.* The stimuli were PBMC medium (Medium) as a background control, Muramyl Dipeptide (MDP) [20 µg/ml], Peptidoglycan-Mono-Tetramer (PGN-Mono-Tet) [6 and 30 µg/ml], L13 [6 and 30 µg/ml] and L15 [6 and 30 µg/ml]. The measured IL-23 concentrations are depicted in pg/ml ± standard error of mean (SEM).

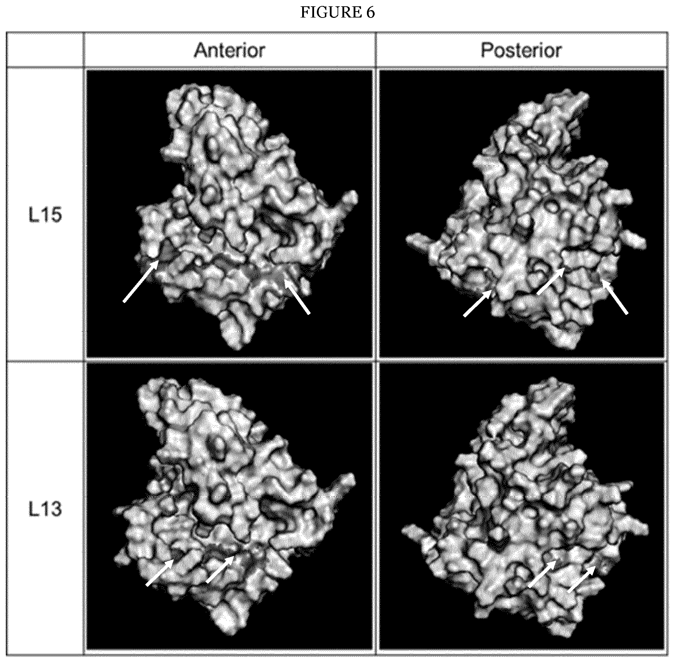

**Figure 6****:** Prediction of Lpli structure and localization of L15 (top row, binding area indicated by white arrows) and L13 (bottom row, binding area indicated by white arrows) in Lpli. 3D structure of Lpli was generated using Robetta program to determine the location of L15 and L13. L15 spanned as 2 β-sheets and 2 loops in the Lpli structure, whereas L13 spanned as a single β-sheet and a loop.

The sequences show (in full length sequences, the position of L15 is BOLD and L13 is underlined):

### Staphylococcus aureus

SEQ ID NO: 1 ABD21538.1 staphylococcal tandem lipoprotein [*Staphylococcus aureus* subsp. aureus USA300_FPR3757] 
MKYKTERREAMGYLKRFALYISVMILIFAIAGCGKGNETKEDSKEEQIKKSFAITLDMYP
IKNLEDLYDKEGYRDSEFKKGDKGMWTIYTDFAKSNKPGELDDEGMVLNLDRNTRTAKGHY
FVTTFYRNGKLPDEKNYKIEMKNNKIILLDEVKDDKLKQKIENFKFFGQYANLKELRKYNNG
DVSINENVPSYDVEYKMSNKDEIVKELRSRYNISTEKSPILKMHIDGDLKGSSVGYRKLEIDFSK
RENSKLSVIEFLSYKPAKK
SEQ ID NO: 2 shows the domain of *S.aureus* Lpli protein: LNC1
   TAKGHYFVTTFYRNGKLPDEKNYKIEMKNNKIILLDEVKDDKLKQKIENFKFFGQYANL KELRK
SEQ ID NO: 3 shows the sequence of the L13 peptide: GHYFVTTFY
SEQ ID NO: 4 shows the sequence of the L15 peptide: TAKGHYFVTTFYRNG

### Staphylococcus hyicus

SEQ ID NO: 5 >VTS35859.1 putative lipoprotein [*Staphylococcus hyicus*]

MMEYIKKIALYMSVLLLIIFIGGCENMKDEQKKEEQTNKTDSKEEQIKKSFAKTLDMYP
IKNLEELYDKEGYRDGEFEKGDKGMWTIYTDFAKSNKPGELNDEGMVLNLDRNTRTAKGHY
FVTTFYRNGKLPDEKNYKIEMKNNKIILLDEVKDDKLKQKIENFKFFGQYANLKELKKYNNG
DVSINENVPSYDVEYKMSNKDEIVKELRSRYNISTEKSPILKMHIDGNLKGSSVGYRKLEIDFSK
RENSKLSVIEFLSYKPAKVNNDER

### Staphylococcus schweitzeri

SEQ ID NO: 6 >CDR53053.1 Staphylococcal tandem lipoprotein [*Staphylococcus schweitzeri*]

MKYKTERREAMGYLKRFALYISVMILIFAIAGCGKGNETKEDSKEEQIKKSFAKTLDMY
PIKNLEDLYDKEGYRDGEFKKGDKGMWTIYTDFAKSNKPGELNDEGMVLNLDRNTRTAKGH
YFVTTFYRNGKLPDEKNYKIEMKNNKIILLDEVKDDKLKQKIENFKFFGQYANLKELKKYNN
GDVSINENVPSYDVEYKMSNKDEIVKELRSSYNISTEKSPILKMHIDGHLKGSSVGYRKLEIDFS
KRENSKLSVIEFLSYKPAKK

### Staphylococcus argenteus

SEQ ID NO: 7 >CDR24818.1 putative lipoprotein [*Staphylococcus argenteus*]

[70] MRTINYDEKRGYGTMNNFRQYALCIGTSVLILLVSGCSGVSDTPEDSKETQIKKSFAKTL
DMYPIKNLEDLYDKEGYRDGEFKKGDKGMWTIYTDFAKSNKPGELNDEGMVLNLDRNTRTA
KGHYFVTTFYRNGKLPDEKNYKIEMKNNKIILLDEVKDDKLKQKIENFKFFGQYANLKELKK
YNNADVSINENVPSYDVEYKMSNKDEIVKELRRRYNISTEKSPILKMHIDGDLKGSSVGYRKLEI
DFSKRENSKLSVIEFLSYKPAKNSIDKEY

### Listeria monocytogenes

SEQ ID NO: 8 >WP_165981412.1 tandem-type lipoprotein, partial [*Listeria monocytogenes*]

NEGMVLYLDRNTRTAKGHYFVKTFYNKGKFPDRKNYKVEMKNNKIILLDKVEDTNL
KKRIENFKFFGQYANLKELKNYNNGDVSINENVPSYDAKFKMSNKDENVKQLRSRYNIPTDKA
PVLKMHIDGNLKGSSVGDRKLEIDFSKRENSHLSVIDSLDYQPAKVDEDER

### Lactobacillus ruminis

SEQ ID NO: 9 >WP_135559033.1 tandem-type lipoprotein, partial [*Lactobacillus ruminis*]

MKSIKRIGLCISLLILSIFVTSCDGDNKITGDSKETQIKKSFSKTLDMYPIKNLEDLYDKEG
YRDSEFKKGDKGMWTIYTDFAKSNKPGELSNEGMVLYLDRNTRTAKGYYFVRTFYRKDKLP
DRKNYKV

### Escherichia coli

SEQ ID NO: 10 >VTR53284.1 Uncharacterized lipoprotein SAV0445 precursor *[Escherichia coli]*

MMGYIKRMALYMSVFLLIIFIVGCRNMKDEQKKEEQTNKTDSKEEQIKKSFEKTLDMY
PIKNLEELYDKEGYRDGEFKKGDKGMWTIYTDFAKSNKQGGLSNEGMVLYLDRNTRTAKGH
YFVKTFYNKGKFPDRKNYKVEMKNNKIILLDKVEDTNLKKRIENFKFFGQYANLKELKNYNN
GDVSINENVPSYDAKFKMSNKDENVKQLRSRYNIPTDKAPVLKMHIDGNLKGSSVGYKKLEID
FSKGGKSDLSVIDSLNFQPAKVDEDDE

### Klebsiella pneumoniae

SEQ ID NO: 11 >WP_171482503.1 tandem-type lipoprotein, partial [*Klebsiella pneumoniae*]

MGYLKRFSLYISVMILIFAIAGCGKGNETKEDSKEEQIKKSFAKTVDMYPIKNLEDLYDK
EGYRDGEFKKGDKGMWTIYTDFAKSNKPGELSNEGMVLYLDRNTRTAKGYYFVRTFYRKDK
LPDRKNYKVEMKNNKI

### Pseudomonas aeruginosa

[84] SEQ ID NO: 12 >MUK59508.1 tandem-type lipoprotein, partial [*Pseudomonas aeruginosa*] 
MEYIKKIALYMSVLLLIIFIGGCENMKDEQKKEEQTNKTDSKEEQIKKSFAKTLDMYPIK
NLEELYDKEGYRDGEFEKGDKGMWTIYTDFAKSNKPGELNDEGMVLNLDRNTRTAKGHYF
VTTFYRNGKLPDEKNYKIEMKNNKIILLDEVKDDKLKQKIENFKFFGQYANLKELKKYNNGD
VSINENVPSYDV
[86] SEQ ID NO: 29: core domain in LPLi *of S. aureus* 
DSKEEQIKKSFAITLDMYPIKNLEDLYDKEGYRDSEFKKGDKGMWTIYTDFAKSNKPGE
LDDEGMVL
[88] SEQ ID NOs: 30-32 show further sequences of Table 1 in the Example section.

Peptide sequences of the invention are shown in **Table A:**

| **Species** | **Peptide 1** | **SEQ ID No of peptide 1** | **Peptide 2** | **SEQ ID No of peptide 2** |
|---|---|---|---|---|
| Staphylococcus hyicus | TAKGHYFVTTFYRNG | 13 | GHYFVTTFY | 21 |
| Staphylococcus schweitzeri | TAKGHYFVTTFYRNG | 14 | GHYFVTTFY | 22 |
| Staphylococcus argenteus | TAKGHYFVTTFYRNG | 15 | GHYFVTTFY | 23 |
| Listeria monocytogenes | TAKGHYFVKTFYNKG | 16 | GHYFVKTFY | 24 |
| Lactobacillus ruminis | TAKGYYFVRTFYR | 17 | GYYFVRTFY | 25 |
| Escherichia coli | TAKGHYFVKTFYNKG | 18 | GHYFVKTFY | 26 |
| Klebsiella pneumoniae | TAKGYYFVRTFYR | 19 | GYYFVRTFY | 27 |
| Pseudomonas aeruginosa | TAKGHYFVTTFYRNG | 20 | GHYFVTTFY | 28 |

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Lpli-derived peptides L13 and L15 are potential drug candidates

*Staphylococcus aureus* lipoproteins (Lpp) represent one of the major classes of surface proteins with roles in immune responses and pathogenicity. In *S. aureus* the lpl genes are part of a pathogenicity island, and lpl-homologous genes are typically aligned in tandem. Since the contribution of the lpl gene cluster to *S. aureus* virulence was unclear, the inventors deleted the entire cluster in *S. aureus* USA300. The mutant showed not only a decrease in the stimulation of pro-inflammatory cytokines in human monocytes, macrophages and keratinocytes. More important were the findings that i) the lpl cluster contributed to invasion *of S. aureus* into human keratinocytes and mouse skin, and ii) the non-invasive *S. carnosus* expressing the lpl gene cluster became invasive. Additionally, in a murine kidney abscess model the bacterial burden in the kidneys was higher in wild type-infected than in mutant-infected mice, indicating that the lpl cluster contributes to virulence (Nguyen et al., 2015).

It was found that it is not the lipid portion but the protein portion alone that is responsible for the host cell invasion. It turned out that the Lpl protein acts as cyclomodulin by causing a delay in the G2/M phase transition (Nguyen et al., 2016). Since it has been observed that bacterial invasion by the host cell occurs mainly in the G2 phase, an extension of the G2 phase might increase the incidence of bacterial invasion.

Indeed, the Lpl protein triggers *S. aureus* internalization by various human epithelial cells and also keratinocytes (Nguyen et al., 2015) via a new mechanism based on the interaction of Lpl with the human heat shock proteins Hsp90α and Hsp90β (Tribelli et al., 2019). Upon interaction Lpli activates Hsp90, which in turn induces F-actin formation and endocytosis-like internalization (Tribelli et al., 2019).

When the sequence of the nine Lpl proteins encoded by the lpl operon in *S. aureus* USA300 was compared, the presence of a conserved core region (92% similarity) (Fig. 1) was observed. This region might be responsible for the activity of Lpli.

To identify Lpli motifs that interact with the target protein Hsp90α the inventors synthesized peptides of various length covering the whole Lpli protein. All the peptides (> 40) were tested for their ability a) to interact with Hsp90α, and b) to trigger *S*. *aureus* internalization by keratinocyte-like HaCaT cells. Most of the peptides had no effect, like SNC1 shown in Table 1.

**Table 1: Selected Lpl1 derived peptides with their effect on USA300 invasion in HaCaT keratinocytes, interaction with Hsp90α and F-actin levels in HaCaT cells**

| **Peptide** | **Sequence** | **Invasion^{a)}** | **Hsp90-interacti on ^{b)}** | **F-actin levels** |
|---|---|---|---|---|
| **Lpl1** | **Native protein (267 aa)** | ⇑ | + | ft |
| LNC1 (64 aa) | TAKGHYFVTTFYRNGKLPDEKNYKIEMK NNKIILLDEVKDDLKQKIENFKFFGQYA NLKELRK | ⇑ | + | ⇑ |
| L13 | GHYFVTTFY | ⇓ | + | ⇓ |
| L15 | TAKGHYFVTTFYRNG | ⇓ | + | ⇓ |
| LNC2 (64 aa) | DEIVKELRSRYNISTEKSPILKMHIDGDLK GSSVGYRKLEIDFSKRENSKLSVIEFLSYK PAKK | ⇑ | + | ⇑ |
| MNC3 (38aa) | RSRYNISTEKSPILKMHIDGDLKGSSVGY RKLEIDFSK | ⇑ | + | ⇑ |
| SNC1 (15aa) | DEIVKELRSRYNIST | no effect | - | no effect |

| | | | | |
|---|---|---|---|---|
| ^{a)} Triggering invasion of USA300 by keratinocytes; ^{b)} Direct interaction with Hsp90α in dot blot assay; ⇑ increased invasion and F-actin level; ⇓ decreased invasion and F-actin level\| | | | | |

5 peptides that interacted with Hsp90α were identified. Three of them (the longest) triggered *S. aureus* internalization by HaCaT cells as did the native Lpl1 protein while other two (L13 and L15) reduced *S. aureus* internalization by HaCaT cells as well as F-actin levels when compared to the full length Lpl1 protein (Table 1).

### Example 2: L13 and L15 showed significant reduction of S. aureus USA300 internalization by HaCaT and N/TERT1 cells

The inventors compared the effect on host internalization of L13 and L15 with that of geldanamycin. Geldanamycin is a well-known cell permeable anti-neoplastic drug that competes with ATP for binding to Hsp90, thus inhibiting Hsp90 activity (Gorska *et al.,* 2012). This in turn leads to inactivation, destabilization, and eventual degradation of Hsp90 client proteins, The geldanamycin analog 17AAG decreased F-actin formation upon Hsp90 inhibition (Taiyab & Rao Ch, 2011). As shown in **Fig. 2** the inhibition of internalization by L13 was almost as efficient as that of geldanamycin; L13 was more efficient than L15.

### Example 3: L13 and L15 are much less cytotoxic than geldanamycin

Cytotoxicity assays were conducted on 5 different mammalian cell lines after 24h and 48h of exposure to the various compounds, using the colorimetric tetrazolium dye MTT and the fluorometric resazurin (Alamar Blue): MM6 (human cell line with characteristics of mature monocytes), HEK293 (human embryonic kidney cells), HaCaT (human keratinocyte cell line), HeLa (human cervical cancer cell line) and HT-29 (human colon cancer cell line). L13 and L15 showed no cytotoxicity (viability) up to a conc. of 100 µM; while geldanamycin showed toxicity already at 10 µM.

### Example 4: L13 and L15 peptide sequences are only found in homologous Lpl-lipoproteins of few other bacterial species.

Using the Basic Local Alignment Search Tool (**BLAST**) the inventors discovered that the amino acid sequence of L15 is also found in lipoproteins from other pathogenic bacteria, including *Listeria monocytogenes, Klebsiella pneumonia* and *Pseudomonas aeruginosa,* but not in mammalian proteins (**Fig. 3A**). The GeneBAnk ID and the identity of L15 sequence is shown in **Fig. 3B****.** Interestingly, the staphylococcal and *P. aeruginosa* L15 sequences are identical, suggesting that Lpl proteins may play a role in virulence not only in *S. aureus* but also in some other pathogens.

### Example 5: L13 and L15 have protective activity in animal models.

The inventors tested L13 and L15 in a systemic infection model with *Galleria mellonella.* The larvae were treated with L13 or L15 and then immediately infected with *S. aureus* USA300. Both peptides significantly decreased the mortality of the larvae (**Fig. 4A**).

Septic arthritis remains a devastating and invasive joint disease. The majority of septic arthritis in patients is caused by hematogenous spreading of bacteria (Mohammad et al., 2020). Encouraged by larvae results we tested the peptides in an approved mouse sepsis model. The septic arthritis model was established as follows: In all experiments, mice are intravenously inoculated with 0.2 ml of the respective bacterial strain suspensions into the tail vein with an arthritogenic dose of 4.0-6.0 106 CFUs per mouse. The mice are individually monitored on a daily basis for survival, weight and the presence of clinical arthritis. In this model the mice that were treated with L15 before application of the bacteria. All mice treated with L15 survived to 100 %, and weight loss was also less dramatic (Fig. 4B). IT was also observed that the mice treated with L15 were hyperactive. The experiment was repeated several times and the results are robust.

### Example 6: L13 induces IL-23, which is crucial for the differentiation of naive T cells to TH17 cells.

The effect of L13 and L15 on the production of cytokines is further investigated on peripheral blood mononuclear cells (PBMCs) from different donors. Using LegendPlex-Kits, the effect of L13 and L15 was investigated on the stimulation/repression of IL-23, IP-10, IL-1RA, IL-12p40, IL-12p70, TNF-α, IL-1β, TARC, IL-6 and IL-10. In contrast to the positive controls Muramyl Dipeptide (MDP) and Peptidoglycan-Mono-Tetramer (PGN-Mono-Tet), which induced high levels of TNF, IL-1β, IL-6 or IL-10, no or only low levels of these cytokines were induced upon stimulation with L13 or L15.

### Example 7: Location of L13 and L15 within Lpli

Intrigued by the finding that shortened peptides show opposite effects compared to the longer native Lpli protein, the inventors used 3D modelling to locate L13 and L15 within Lpli. Both peptides (L13 is part of L15) are located within the β-sheet region that is mostly unexposed to the exterior surface (Fig. 6). The location suggests that, in the context of the full-length protein, L15 and L13 might be unavailable for binding to Hsp90α. The inventors do not believe that Lpli after binding to Hsp90 undergoes a conformational change in such a way that L15 sequence becomes exposed, because Lpli activates Hsp90 while synthetic L13 and L15 inhibit it (Table 1). It is however assumed that Lpli binds to Hsp90α at a site where it causes activation of this receptor, while L13 and L15 interact with Hsp90α at an allosteric site that inhibits Hsp90α. More studies are required to understand the molecular basis of inhibition and activation by L13-L15 and Lpli, respectively.

It was also investigated whether the peptides might be produced in *vivo* by proteolytic digestion with proteases. *In silico* analysis using the Peptide Cutter program, predicts that the peptides are not generated by proteolytic degradation.

### REFERENCES

The most relevant references are:
1. Danielyan, L., Schwab, M., Siegel, G., Brawek, B., Garaschuk, O., Asavapanumas, N., Buadze, M., Lourhmati, A., Wendel, H.P., Avci-Adali, M., Krueger, M.A., Calaminus, C., Naumann, U., Winter, S., Schaeffeler, E., Spogis, A., Beer-Hammer, S., Neher, J,J., Spohn, G., Kretschmer, A., Krämer-Albers, E.M., Barth, K., Lee, H.J., Kim, S.U., Frey, W.H. 2nd, Claussen, C.D., Hermann, D.M., Doeppner, T.R., Seifried, E., Gleiter, C.H., Northoff, H., Schäfer, R. (2020) Cell motility and migration as determinants of stem cell efficacy. EBioMedicine 60: 102989. doi: 10.1016/j.ebiom.2020.102989. Epub 2020 Sep 10.
2. Vega, S.C., Leiss, V., Piekorz, R., Calaminus, C., Pexa, K., Vuozzo, M., Schmid, A.M., Devanathan, V., Kesenheimer, C., Pichler, B.J., Beer-Hammer, S., Nürnberg, B. (2020) Selective protection of murine cerebral Gi/o-proteins from inactivation by parenterally injected pertussis toxin. J Mol Med 98(1): 97-110. doi: 10.1007/s00109-019-01854-1. Epub 2019 Dec 6.
3. Arefanian, S., Schäll, D., Chang, S., Ghasemi, R., Higashikubo, R., Zheleznyak, A., Guo, Y., Yu, J., Asgharian, H., Li, W., Gelman, A.E., Kreisel, D., French, A.R., Zaher, H., Plougastel-Douglas, B., Maggi, L., Yokoyama, W., Beer-Hammer, S.*, Krupnick, A.S.* (2016) Deficiency of the Adaptor Protein SLy1 Results in a Natural Killer Cell Ribosomopathy Affecting Tumor Clearance. OncoImmunology 5(12): e1238543*.* *shared last author
4. Mauriac, S., Hien, Y., Bird, J. et al. Defective Gpsm2/Gαi3 signalling disrupts stereocilia development and growth cone actin dynamics in Chudley-McCullough syndrome. Nat Commun 8, 14907 (2017). https://doi.org/10.1038/ncomms14907.
5. Autenrieth, S.E., Warnke, P., Wabnitz, G.H., Lucero Estrada, C., Pasquevich, K.A., Drechsler, D., Günter, M., Hochweller, K., Novakovic, A., Beer-Hammer, S., Samstag, Y., Hämmerling, G.J., Garbi, N., Autenrieth, I.B. (2012) Depletion of dendritic cells enhances innate antibacterial host defense through modulation of phagocyte homeostasis. PLOS Pathogen 8, e1002552.

## Claims

1. **An isolated peptide, or a peptide-derivative or -analog thereof,** wherein the peptide sequence has a length of 9 to 40 amino acids, and wherein the peptide sequence comprises, or preferably consists essentially of, or consists of, a consecutive sequence of the formula: X₁X₂X₃X₄X₅X₆X₇X₈X₉X₁₀X₁₁X₁₂X₁₃X₁₄X₁₅, wherein X is an amino acid or no amino acid (absent) according to the following definitions:
- X₁ is absent or T, preferably X₁ is absent if X₂ is absent;
- X₂ is absent or A, preferably X₂ is absent if X₃ is absent;
- X₃ is absent or K;
- X₄ is G;
- X₅ is any amino acid, preferably any amino acid is Y or H;
- X₆ is Y;
- X₇ is F;
- X₈ is V;
- X₉ is any amino acid, preferably any amino acid is T, K or R;
- X₁₀ is T;
- X₁₁ is F;
- X₁₂ is Y;
- X₁₃ is absent or any amino acid, preferably any amino acid is R or N;
- X₁₄ is absent or any amino acid (preferably any amino acid is N or K), preferably X₁₄ is absent if X₁₃ is absent;
- X₁₅ is absent or any amino acid (preferably any amino acid is G or D), preferably X₁₅ is absent if X₁₄ is absent.
- X₁₅ is absent or any amino acid.

2. The isolated peptide of claim 1 wherein the peptide sequence has a length of 9 to 30 amino acids, preferably of 9 to 20 amino acids, most preferably of 9 to 16 amino acids.

3. The isolated peptide of claim 1 or 2, comprising, or consisting essentially of, a sequence that is at least 90% identical, preferably 100% identical, to a peptide shown in any of SEQ ID No: 3, 4 and 13 to 28.

4. The isolated peptide of claim 1 or 2, comprising, or consisting essentially of, a sequence that is at least 90% identical, preferably 100% identical, to GHYFVTTFY or TAKGHYFVTTFYRNG (SEQ ID NO: 3 or 4).

5. The isolated peptide of any one of claims 1 to 4, wherein the peptide has a maximal length of 16 amino acids, preferably of 15 amino acids, preferably wherein the length of the isolated peptide consists of either 9 or 15 amino acids.

6. The isolated peptide of any one of claims 1 to 5 wherein the peptide, or the peptide-derivative or -analog thereof, comprises an N-terminal formylation, and/or a C-terminal amidation.

7. **A fusion peptide or fusion protein** consisting of two sequence portions, wherein the first sequence portion is identical to the sequence of the isolated peptide recited in any one of claims 1 to 6, and wherein the second portion consists of a sequence that is not more than 70%, preferably 50% even more preferably 30% identical to sequence of similar length in a bacterial lipoprotein.

8. **An isolated nucleic acid** comprising a nucleic acid sequence encoding for a peptide or protein, wherein the encoded peptide or protein consists of a peptide of any one of claims 1 to 6 or consists of a fusion peptide or fusion protein of claim 7.

9. **A vector** comprising the nucleic acid of claim 8.

10. **A recombinant cell** comprising the nucleic acid of claim 8, or the vector of claim 9.

11. **A pharmaceutical composition** comprising (i) the isolated peptide of any one of claims 1 to 6, or the fusion peptide or fusion protein of claim 7, or the nucleic acid of claim 8, or the vector of claim 9, or the recombinant cell of claim 10; and (ii) a pharmaceutically acceptable carrier and/or excipient.

12. **A compound or composition for use in the treatment of an infection** in a subject, wherein the compound or composition comprises the isolated peptide of any one of claims 1 to 6, or the fusion peptide or fusion protein of claim 7, or the nucleic acid of claim 8, or the vector of claim 9, or the recombinant cell of claim 10, or the pharmaceutical composition of claim 11.

13. The compound or composition for use of claim 12, wherein the compound or composition when administered (i) inhibits bacterial internalization in one or more host cells, and/or (ii) enhances the hosts immune reaction to the bacterial infection.

14. The compound or composition for use of claim 12 or 13, wherein the compound or composition comprises one or more further therapeutically active ingredients, such as preferably one or more antibiotics.

15. The compound or composition for use of claim 12 or 13, wherein the compound or composition comprises one or more further therapeutically active ingredients, such as cell proliferation stimulating compounds.
